# EUROPEAN PATENT APPLICATION

(11) **EP 3 699 286 A1**
(43) Date of publication of application: **26.08.2020**
(21) Application number: 19212363.6
(22) Date of filing: 20.04.2012
(51) Int. Cl.: C12N 15/861, A61K 39/40, A61K 39/42

(54) **REGIMENS AND COMPOSITIONS FOR AAV-MEDIATED PASSIVE IMMUNIZATION OF AIRBORNE PATHOGENS**

(30) Priority: 20.04.2011 US 201161477454 P; 06.03.2012 US 201261607196 P
(62) Divisional of application: 12719540.2
(71) Applicant: The Trustees of the University of Pennsylvania, Philadelphia, PA 19104-3242 (US)
(72) Inventor: WILSON, James M., Philadelphia, PA 19103 (US); LIMBERIS, Maria P., Philadelphia, PA 19103 (US)
(74) Representative: Manaton, Ross Timothy

(57) **Abstract**

A prophylactic regimen for passively preventing infection with a pathogen which has a typical route of infection through the nasopharynx region of a subject, e.g., an airborne virus typically transmitted through coughing or sneezing. The method involves specifically targeting a subject's nasopharynx with a viral vector comprising an AAV capsid and carrying a nucleic acid sequence encoding an anti-viral neutralizing antibody construct operably linked to expression control sequences, in order to provide for high levels of expression of the anti-viral neutralizing antibody construct in the nasal airway cells. Optionally, the neutralizing antibody construct is expressed under a promoter which is regulated or induced by a small molecule which is delivered separately from the viral vector. In one embodiment, the method permits transfection of a subject's nasopharynx even where the subject has circulating neutralizing antibodies against the AAV capsid.

## Description

### BACKGROUND OF THE INVENTION

In the USA, influenza (flu) is the seventh leading cause of death. The young, elderly and pregnant women are most at risk. In 2009, the H1N1 pandemic strain affected almost 60 million people and resulted in ∼250,000 hospitalizations. In the last century there were three pandemics, including the 1918 pandemic flu that killed tens of millions of people. It is expected that another flu pandemic will occur in the 21st century. The traditional prophylactic vaccines for epidemic flu are produced well in advance of the flu season and they are based on a hypothetical and expected strain of flu. However, the effectiveness of the flu vaccine is widely compromised by the unpredictable appearance of new subtypes possessing distinct and unique surface antigens from those that are present in the currently circulating influenza viruses. Small molecule drugs such as neuraminidase inhibitors, oseltamivir, amarmivir and amantadine are not effective at controlling the spread of disease and may have contributed to the increased numbers of resistant influenza viruses.

Influenza A viruses, in particular human H3N2 and H1N1 subtypes, are responsible for most infections. The natural adaptive immune response to influenza involves a humoral immune response, with neutralizing antibodies (NAbs) generated against the highly immunogenic haemagglutinin (HA) protein, as well as a cellular immune response. Although protective, NAbs also exert selective pressure resulting in antigenic drift which gives rise to new antigenic variants and allows influenza to escape established immunity.

Recently, novel monoclonal antibodies (mAbs) with broad neutralizing capacity have been identified using antibody phage display to screen libraries from a) donors recently vaccinated with the seasonal flu vaccine, b) non-immune humans or c) survivors of H5N1 infection. These antibodies were shown to neutralize more than one influenza subtype by blocking viral fusion with the host cell. However, many mAbs offer only serotype-restricted protection and are costly to produce, due in part to being limited by the source of polyclonal antibodies.

While rarely seen in Western countries, Ebola outbreaks regularly affect communities/tribes in Central Africa often with devastating consequences. The unpredicted outbreak of severe acute respiratory syndrome (SARS) in China in 2003 resulted in approximately 800 deaths and sickened more than 8,000 people worldwide. Many prophylactic vaccine regimens for Ebola and SARS have focused on conferring protection by systemic immunization.

There continues to be a need for effective methods for conferring protection against potentially pandemic viruses.

### SUMMARY OF THE INVENTION

The invention provides a regimen for passively preventing infection with a pathogen which has a typical route of infection through the nasopharynx region of a subject, *e.g.,* an airborne virus, toxin, bacterium, or other pathogen. The method involves specifically targeting a subject's nasopharynx with an AAV vector carrying a nucleic acid sequence encoding an anti-pathogen construct operably linked to expression control sequences, in order to provide for high levels of expression of the anti-pathogen antibody construct in the nasal airway cells. The regimen involves delivering a composition comprising an effective amount of the AAV viral vectors intranasally such that a therapeutically effective amount is delivered to the nasopharynx in the absence of any therapeutically significant expression in the lung. In one embodiment, the delivery of low volume AAV restricts more than about 70%, more than 80%, more than 90%, more than about 95%, or more than about 99% of expression to the nasal epithelium where a constitutive promoter is utilized. Use of a tissue-specific, cell specific, or an inducible or regulatable promoter delivered locally to the nose further limits expression to the nasal/nasopharynx epithelium. In one embodiment, the subject's nasopharynx cells are transduced even in the presence of high level serum-circulating AAV neutralizing antibodies.

In one embodiment, the anti-pathogen construct is a neutralizing antibody construct. In another embodiment, the regulatory sequences comprise a promoter which is regulated or induced by a small molecule which is delivered separately from the viral vector. In one embodiment, the AAV-mediated delivery to the nasopharynx region transfects the subject even where the subject has circulating neutralizing antibodies against the AAV capsid.

In one embodiment, the invention provides a passive immunization regimen for an airborne pathogen, said regimen comprising specifically expressing anti-pathogen constructs in a subject's nasopharnyx cells by delivering to said cells a composition comprising an AAV viral vector comprising a nucleic acid sequence encoding an anti-pathogen construct operably linked to expression control sequences. The AAV vector may comprise a regulatable promoter which directs expression of an anti-pathogen construct following induction by a ligand for the promoter (*e.g.*, a small molecule compound). In one embodiment, the small molecule compound is rapamycin or a rapamycin analog ("a rapalog").

In one embodiment, the airborne pathogen is a pathogenic virus and the anti-pathogen constructs are neutralizing antibody constructs directed against said virus. The pathogenic virus may be a virus associated with a pandemic, *e.g.*, influenza, Ebola virus, and severe acute respiratory syndrome (SARS). In another embodiment, the airborne pathogen is a bacterium or a bacterial toxin and the anti-pathogen constructs are anti-microbial constructs directed against said bacteria or a pathogenic toxin thereof.

In one aspect, the neutralizing antibody construct is selected from the group consisting of a full-length antibody, a single chain antibody, a Fab fragment, a univalent antibody, and an immunoadhesin. In one embodiment, the neutralizing antibody construct is a monoclonal antibody.

In another aspect, the regimen involves delivering a combination of AAV vectors which comprise different anti-pathogen constructs.

In one embodiment, the AAV vectors are based on an AAV capsid selected from one or more of AAV1, AAV2, AAV3, AAV4, AAV5, AAV6, AAV6.2, AAV7, AAV8, AAV9, rh10, rh64R1, rh64R2 or rh8. In one embodiment, the AAV vector is an AAV9 vector.

Still other aspects and advantages of the invention will be readily apparent to one of skill in the art from the following detailed description of the invention.

### BRIEF DESCRIPTION OF THE FIGURES

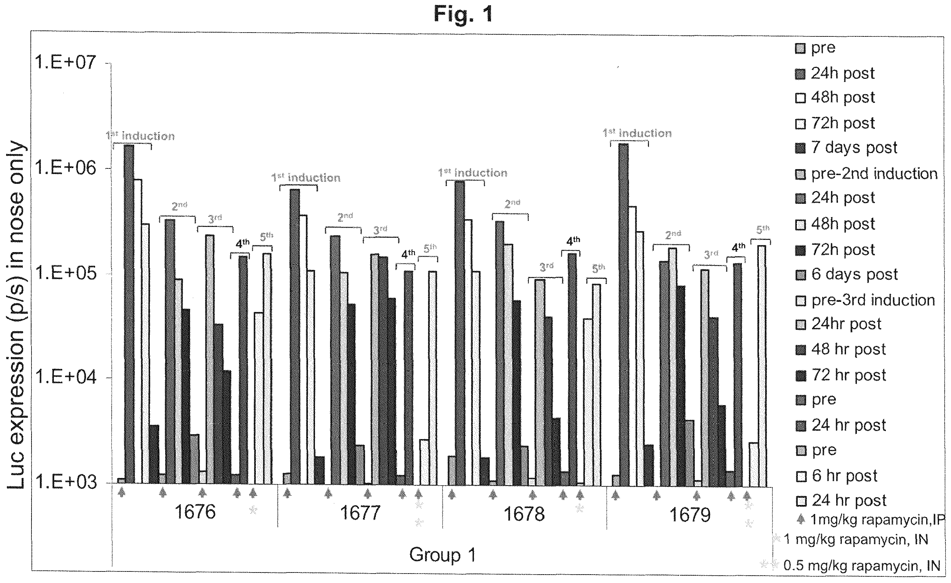

Fig. 1 illustrates the kinetics of luc expression in the nose in mice injected with both the AAV9 vector expressing luc and the AAV9 vector expressing transcription factor (tf) following each of the five rapamycin inductions. The fifth and last induction was performed using rapamycin delivered intranasally.

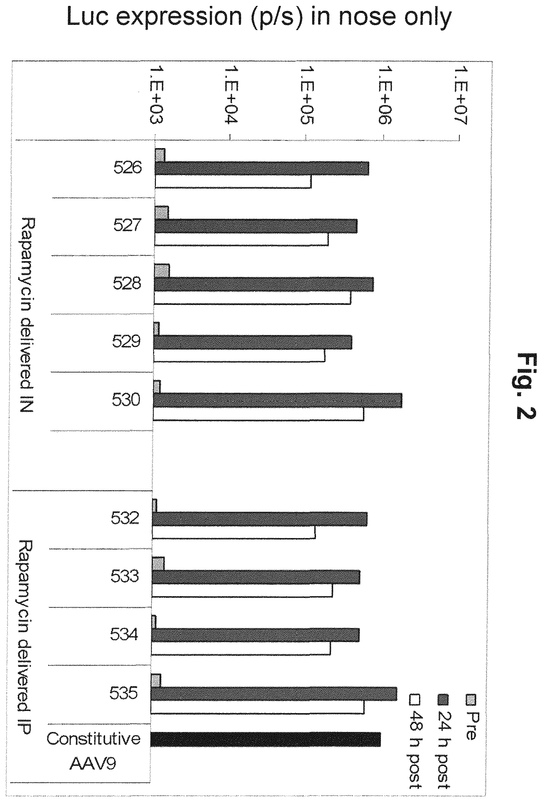

Fig. 2 illustrates fLuc expression in the nose of mice receiving co-injections of an AAV9 inducible vector expressing luc and an AAV9 vector expressing Tf pre and post-rapamycin induction delivered intranasally or intraperitoneally. The black bar is the average expression (1.1 x 10⁶ ± 5.7 x 10⁵ photons/sec) of 9 mice expressing Luc following intranasal delivery of the constitutive AAV9 vector. This study demonstrates that the inducible vector can achieve levels of gene expression similar to the constitutive AAV9 vector.

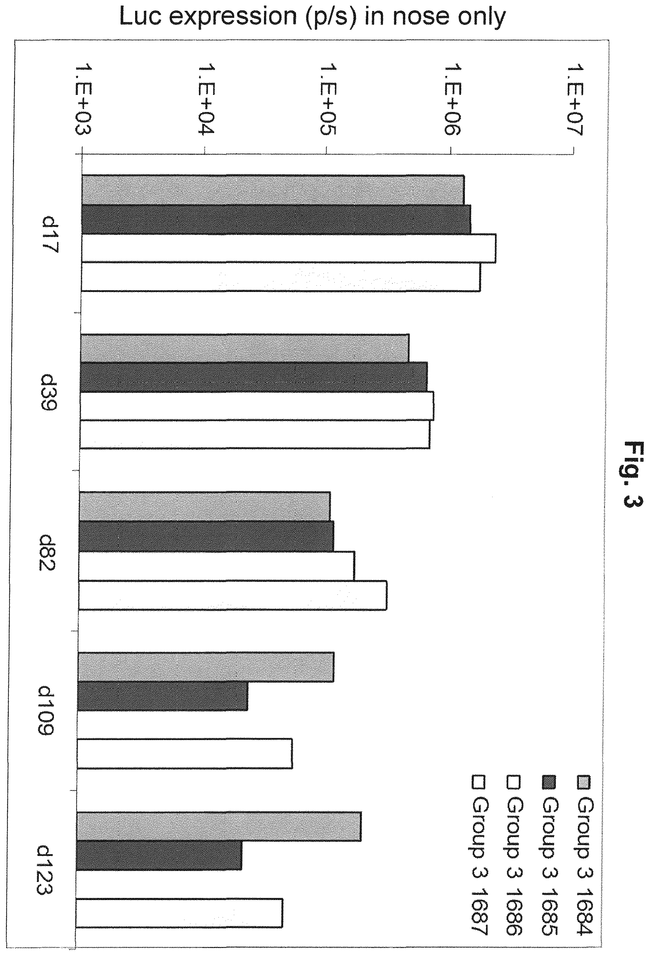

Fig. 3 illustrates long-term gene expression from a constitutive AAV9 vector in the nose.

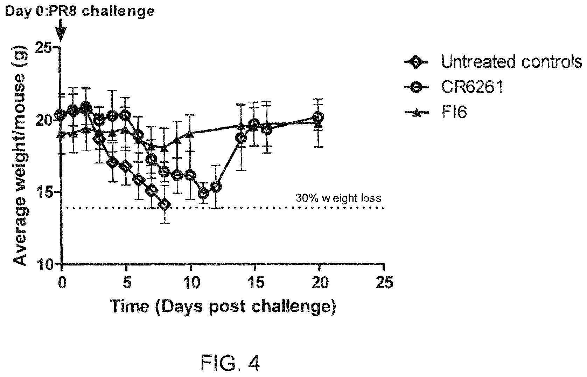

Fig. 4 illustrates results of a study when female BalbC mice were given 2X10¹¹ genome copies of AAV2/9 expressing an CR6261 antibody or an FI6 antibody and challenged 14 days later (noted as day 0 on the graph) with a lethal dose of an influenza virus. Naive mice died within 8 days of exposure while AAV2/9.CB.CR6261 or AAV2/9.CB.FI6 treated mice recovered from significant weight loss with 4/6 and 6/6 mice, respectively, surviving.

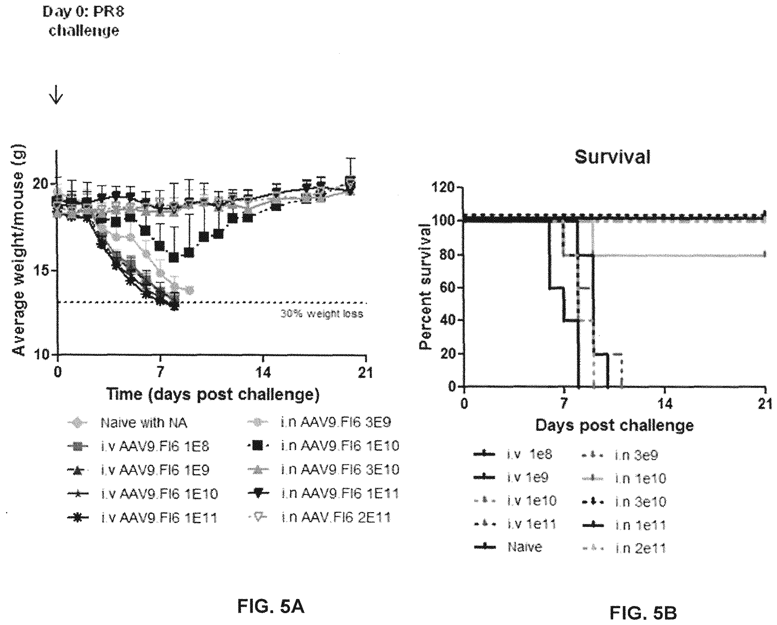

Figs. 5A and 5B show the results of a study evaluating the minimal effective dose of AAV2/9 vector expressing F16 antibody (Ab) following neuraminidase pretreatment. Fig. 5A provides the weight of influenza-challenged mice with time. Mice were euthanized at ≤30% weight loss. Fig. 5B provides a Kaplan Meier survival analysis of influenza-challenged naïve and vector-treated mice.

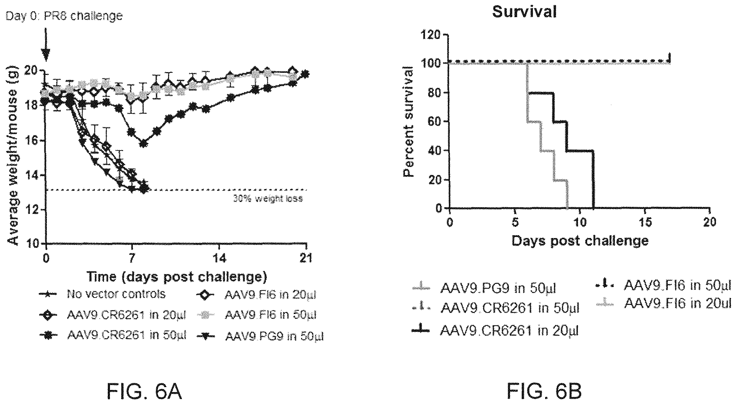

Figs. 6 and 6B provide the results of a study evaluating the protective efficacy of the AAV2/9.CB7.CR6261 or AAV2/9.CB7.FI6 vector delivered to the lung or nose. Fig. 6A provides the weight of influenza-challenged mice with time. Mice were euthanized at ≤30% weight loss. Fig. 6B is a Kaplan Meier survival analysis of influenza-challenged naïve and vector-treated mice.

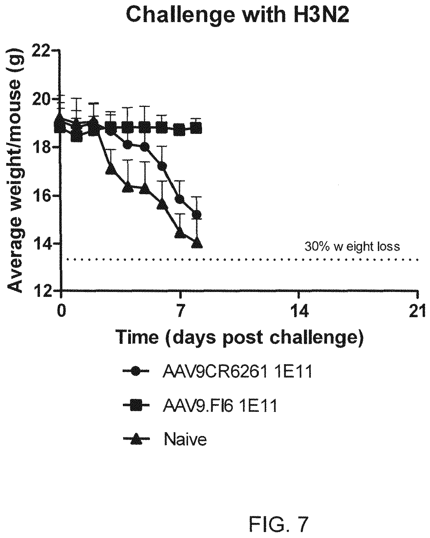

Fig. 7 provides the results of a study assessing the protective efficacy of the AAV-Ab vector against a multi-influenza strain . This graph shows the change in weight of influenza-challenged mice with time. Mice were euthanized at ≤30% weight loss..

### DETAILED DESCRIPTION OF THE INVENTION

The present invention provides a method for generating a passive immunization regimen. The invention utilizes AAV vectors engineered to express an anti-pathogenic construct. Delivery of these AAV vectors to the nose results in high concentrations of neutralizing antibody construct in the airway surface fluid in the nasopharynx. This passive vaccine can then be administered in a non-invasive painless manner, directly to the nose, in an open field and could in fact be self-administered. Additionally, in one embodiment, a bank of passive vaccines can be readily generated and used from broadly neutralizing antibodies.

In the present invention, the viral vectors are delivered locally to the nose. In one embodiment, a composition containing the viral vectors encoding the anti-pathogen construct (*e.g.*, a neutralizing antibody construct) is delivered intranasally as liquid (*e.g.*, atomized, aerosol, spray, etc), to the subject at a relatively low instillation volume in order to minimize lung transduction. In one embodiment, the delivery of low volume AAV restricts more than about 70%, more than 80%, more than 90%, more than about 95%, or more than about 99% of transduction (as measured by expression) to the nasal epithelium. In another embodiment, the viral vectors may be delivered locally through a means other than an intranasal spray, *e.g.*, intranasal injection.

In one embodiment, a pharmacologically regulatable promoter permits further control of the expression of the anti-pathogen construct (s), through controlling (optionally in a dose dependent manner) the contact between the pharmacologic agent and the nasal/nasopharynx cells carrying the AAV carrying the coding sequences for the anti-pathogen construct(s). More particularly, the ligand for the regulatable promoter (*e.g.,* a pharmacologic agent such as rapamycin or a rapalog) may be delivered locally to the AAV-transfected cells of the nasopharynx. This local delivery may be by intranasal injection. However, in another embodiment, the inventors have found that the rapamycin or rapalog is capable of regulating expression if delivered topically to the cells. Topical delivery may be by delivering a bolus containing the rapamycin or rapalog to each nostril/nare. In another embodiment, topical delivery may be accomplished by formulating the rapamycin or rapalog in a suitable composition for topical delivery (*e.g.*, a cream or gel). In still another embodiment, the compositions and methods may be adapted for use with another ligand for anti-pathogen constructs which are under the control of a different regulatable system. Such a pharmacologically-regulated AAV vector provides improved safety, as the anti-pathogenic construct would only be expressed for a few days at a time that is critical for the control of disease severity and spread of the infection. Expression will diminish with time to return to background levels (*e.g.*, within about ~5 days, although expression in some systems may persist for a shorter time (*e.g.,* 3 or 4 days) or for a longer time (*e.g.*, 6, 7, 8, 9 or 10 days), thus greatly minimizing the risk of immune complex formation.

Additionally, the diminution of expression with time is another safety advantage since the nasal cells that harbor the AAV vector will be turned over with time, minimizing any concerns that may be associated with (a) the persistence of expression in the airway or (b) the presence of AAV vector genomes in the target cell. The turnover rate of the nasal airway epithelium is considered to be approximately three months.

### Definitions

The invention confers passive immunity against an airborne pathogen, which as used herein includes an infectious agent which typically infects through cells in the nasal or/or nasopharynx region of a subject. Such pathogens may include, *e.g.,* viruses, bacteria and/or bacterial toxins, and fungi. In addition to selecting agents which are disease-causing for humans, the invention may also be used in passive immunization against veterinary diseases in non-human mammals, including, *e.g*., horses, livestock such as cattle, sheep, goats, swine, etc., amongst others.

The compositions and methods of the invention are designed to provide for delivery of anti-pathogenic constructs to nasal cells and/or the cells of the nasopharynx. The nasopharynx is the nasal part of the pharynx which lies behind the nose and above the level of the soft palate and which is believed to be the primary site of infection from naturally acquired respiratory infections, *e.g*., the as flu. These target nasal and nasopharynx cells include nasal epithelial cells, which may be ciliated nasal epithelial cells, microvilli coated columnar epithelial cells, goblet cells (which secrete mucous onto the surface of the nasal cavity which is composed of the ciliated and microvilli coated cells), and stratified squamous nasal epithelial cells which line the surface of the nasopharynx. In one embodiment, an AAV viral vector is engineered to contain a promoter which is specific for only a subset of these cells (*e.g.*, only the ciliated nasal epithelial cells). In another embodiment, an AAV viral vector is delivered via a route which specifically targets one or more of these cell types, *e.g.*, by delivery of a liquid at a volume which is sufficiently low to preclude any significant update vector by the lung. In one embodiment, an inducible or regulatable promoter is utilized and the inducing or regulating agent is delivered locally to the nasal and/or nasopharynx region. Still other methods for selectively expressing the anti-pathogenic constructs in these cells will be described herein.

An "anti-pathogen construct" is a protein, peptide, or other molecule encoded by a nucleic acid sequence carried on a viral vector as described herein, which is capable of providing passive immunity against the selected pathogenic agent or a cross-reactive strain of the pathogenic agent. In one embodiment, the anti-pathogen construct is a neutralizing antibody construct against the pathogenic agent, *e.g.*, a virus, bacterium, fungus, or a pathogenic toxin of said agent (*e.g*., anthrax toxin).

A "neutralizing antibody" is an antibody which defends a cell from an antigen or infectious body by inhibiting or neutralizing its biological effect. In one embodiment, "neutralizes" and grammatical variations thereof, refer to an activity of an antibody that prevents entry or translocation of the pathogen into the cytoplasm of a cell susceptible to infection. As used herein a "neutralizing antibody construct" includes a full-length antibody (an immunoglobulin molecule), as well as antibody fragments or artificial constructs which have the ability to inhibit or neutralize an antigen or infectious agent. These antibody fragments or artificial constructs may include a single chain antibody, a Fab fragment, a univalent antibody, or an immunoadhesin. The neutralizing antibody construct may be a monoclonal antibody, a "humanized" antibody, a polyclonal antibody, or another suitable construct.

An "immunoglobulin molecule" is a protein containing the immunologically-active portions of an immunoglobulin heavy chain and immunoglobulin light chain covalently coupled together and capable of specifically combining with antigen. Immunoglobulin molecules are of any type (*e.g.,* IgG, IgE, IgM, IgD, IgA and IgY), class (*e.g.*, IgG1, IgG2, IgG3, IgG4, IgA1 and IgA2) or subclass. The terms "antibody" and "immunoglobulin" may be used interchangeably herein.

An "immunoglobulin heavy chain" is a polypeptide that contains at least a portion of the antigen binding domain of an immunoglobulin and at least a portion of a variable region of an immunoglobulin heavy chain or at least a portion of a constant region of an immunoglobulin heavy chain. Thus, the immunoglobulin derived heavy chain has significant regions of amino acid sequence homology with a member of the immunoglobulin gene superfamily. For example, the heavy chain in a Fab fragment is an immunoglobulin-derived heavy chain.

An "immunoglobulin light chain" is a polypeptide that contains at least a portion of the antigen binding domain of an immunoglobulin and at least a portion of the variable region or at least a portion of a constant region of an immunoglobulin light chain. Thus, the immunoglobulin-derived light chain has significant regions of amino acid homology with a member of the immunoglobulin gene superfamily.

An "immunoadhesin" is a chimeric, antibody-like molecule that combines the functional domain of a binding protein, usually a receptor, ligand, or cell-adhesion molecule, with immunoglobulin constant domains, usually including the hinge and Fc regions.

A ""fragment antigen-binding" (Fab) fragment" is a region on an antibody that binds to antigens. It is composed of one constant and one variable domain of each of the heavy and the light chain.

In another embodiment, the invention permits delivery of a nucleic acid construct to the nasopharynx which can be used to "knock-in", "knock-out" or "knock-down" a gene, *e.g.*, for treatment of diseases associated with the nasopharyngeal region, including, *e.g.*, nasopharyngeal cancer, sinusitis, tonsillitis, allergic rhinitis, etc. In yet another embodiment, the invention may be used as a tool in animal models to allow for identification of novel therapeutic targets for diseases associated with the nasopharynx.

"Knockout gene therapy" is directed towards the products of oncogenes (genes that can stimulate the formation of a cancerous tumor). The goal in this type of gene therapy is to render the cancerous cells inactive, while also decreasing the growth of cancerous cells.

"Knockdown gene therapy" is directed towards a gene product which is associated with a disease or conditions in which the targeted gene is overexpressed, but which is not entirely extinguished by the therapy. Molecules such as microRNA and small interfering RNA (siRNA) may be delivered to accomplish knock out or knock down.

An adeno-associated virus (AAV) viral vector is an AAV DNase-resistant particle having an AAV protein capsid into which is packaged nucleic acid sequences for delivery to target cells. In one embodiment, the AAV sequences on the expression cassette comprise only minimal AAV sequences to avoid the risk of replication. In one embodiment, the minimal AAV sequences include the AAV inverted terminal repeat sequences (ITR). In one embodiment, the 5' ITR and the 3' ITR are the minimal AAV sequences required *in cis* in order to express a transgene encoded by a nucleic acid sequence packaged in the AAV capsid. Typically, the ITRs flank the coding sequence for a selected gene product, e.g., an anti-pathogenic construct. In one embodiment, the AAV vector contains AAV 5' and 3' ITRs, which may be of the same AAV origin as the capsid, or which of a different AAV origin (to produce an AAV pseudotype). For example, a pseudotyped AAV illustrated in the examples below contains an expression cassette comprising AAV2 ITRs packaged in an AAV9 capsid. In one embodiment, the coding sequences for the replication (rep) and/or capsid (cap) are removed from the AAV genome and supplied *in trans* or by a packaging cell line in order to generate the AAV vector.

An AAV capsid is composed of 60 capsid protein subunits, VP1, VP2, and VP3, that are arranged in an icosahedral symmetry in a ratio of 1:1:10. Tissue specificity is determined by the capsid type. For example, a viral vector having an AAV9 capsid is illustrated in the examples below as being useful for transducing nasal epithelial cells. The sequences of AAV9 have been described, as have methods of generating vectors having the AAV9 capsid and chimeric capsids derived from AAV9. *See, e.g.,* US 7,906,111, which is incorporated by reference herein. Other AAV serotypes which transduce nasal cells may be selected as sources for capsids of AAV viral vectors (DNase resistant viral particles) including, *e.g.*, AAV1, AAV2, AAV3, AAV4, AAV5, AAV6, AAV6.2, AAV7, AAV8, AAV9, rh10, AA Vrh64R1, AAVrh64R2, rh8 [See, e.g., US Published Patent Application No. 2007-0036760-A1; US Published Patent Application No. 2009-0197338-A1; EP 1310571]. *See also*, WO 2003/042397 (AAV7 and other simian AAV), US Patent 7790449 and US Patent 7282199 (AAV8), WO 2005/033321 (AAV9), and WO 2006/110689], or yet to be discovered, or a recombinant AAV based thereon, may be used as a source for the AAV capsid. These documents also describe other AAV which may be selected for generating AAV and are incorporated by reference. In some embodiments, an AAV cap for use in the viral vector can be generated by mutagenesis *(i.e.,* by insertions, deletions, or substitutions) of one of the aforementioned AAV Caps or its encoding nucleic acid. In some embodiments, the AAV capsid is chimeric, comprising domains from two or three or four or more of the aforementioned AAV capsid proteins. In some embodiments, the AAV capsid is a mosaic of Vpl, Vp2, and Vp3 monomers from two or three different AAVs or recombinant AAVs. In some embodiments, an rAAV composition comprises more than one of the aforementioned Caps.

In some embodiments, an AAV capsid for use in an rAAV composition is engineered to contain a heterologous sequence or other modification. For example, a peptide or protein sequence that confers selective targeting or immune evasion may be engineered into a capsid protein. Alternatively or in addition, the capsid may be chemically modified so that the surface of the rAAV is polyethylene glycolated (PEGylated). The capsid protein may also be mutagenized, *e.g.,* to remove its natural receptor binding, or to mask an immunogenic epitope.

The AAV viral vector contains a nucleic acid sequence encoding an anti-pathogenic construct under the control of regulatory sequences which control transcription and/or expression of the anti-pathogenic construct. Expression control or regulatory sequences may include, e.g., include appropriate transcription initiation, termination, promoter and enhancer sequences; efficient RNA processing signals such as splicing and polyadenylation (polyA) signals; sequences that stabilize cytoplasmic mRNA; sequences that enhance translation efficiency (*i.e.*, Kozak consensus sequence); sequences that enhance protein stability; and when desired, sequences that enhance secretion of the encoded product. A promoter may be selected from amongst a constitutive promoter, a tissue-specific promoter, a cell-specific promoter, a promoter responsive to physiologic cues, or an inducible promoter.

Examples of constitutive promoters suitable for controlling expression of the therapeutic products include, but are not limited to chicken β-actin (CB) promoter, human cytomegalovirus (CMV) promoter, the early and late promoters of simian virus 40 (SV40), U6 promoter, metallothionein promoters, EF1α promoter, ubiquitin promoter, hypoxanthine phosphoribosyl transferase (HPRT) promoter, dihydrofolate reductase (DHFR) promoter (Scharfmann et al., Proc. Natl. Acad. Sci. USA 88:4626-4630 (1991), adenosine deaminase promoter, phosphoglycerol kinase (PGK) promoter, pyruvate kinase promoter phosphoglycerol mutase promoter, the β-actin promoter (Lai et al., Proc. Natl. Acad. Sci. USA 86: 10006-10010 (1989), the long terminal repeats (LTR) of Moloney Leukemia Virus and other retroviruses, the thymidine kinase promoter of Herpes Simplex Virus and other constitutive promoters known to those of skill in the art. Examples of tissue- or cell-specific promoters suitable for use in the present invention include, but are not limited to, endothelin-I (ET -I) and Flt-I, which are specific for endothelial cells, FoxJ1 (that targets ciliated cells).

Inducible promoters suitable for controlling expression of the therapeutic product include promoters responsive to exogenous agents (*e.g.*, pharmacological agents) or to physiological cues. These response elements include, but are not limited to a hypoxia response element (HRE) that binds HIF-Iα and *β*, a metal-ion response element such as described by Mayo et al. (1982, Cell 29:99-108); Brinster et al. (1982, Nature 296:39-42) and Searle et al. (1985, Mol. Cell. Biol. 5:1480-1489); or a heat shock response element such as described by Nouer et al. (in: Heat Shock Response, ed. Nouer, L., CRC, Boca Raton, Fla., ppI67-220, 1991)

In one embodiment, expression of the neutralizing antibody construct is be controlled by a regulatable promoter that provides tight control over the transcription of the gene encoding the neutralizing antibody construct, *e.g.,* a pharmacological agent, or transcription factors activated by a pharmacological agent or in alternative embodiments, physiological cues. Promoter systems that are non-leaky and that can be tightly controlled are preferred.

Examples of regulatable promoters which are ligand-dependent transcription factor complexes that may be used in the invention include, without limitation, members of the nuclear receptor superfamily activated by their respective ligands (e.g., glucocorticoid, estrogen, progestin, retinoid, ecdysone, and analogs and mimetics thereof) and rTTA activated by tetracycline. In one aspect of the invention, the gene switch is an EcR-based gene switch. Examples of such systems include, without limitation, the systems described in US Patent Nos. 6,258,603, 7,045,315, U.S. Published Patent Application Nos. 2006/0014711, 2007/0161086, and International Published Application No. WO 01/70816. Examples of chimeric ecdysone receptor systems are described in U.S. Pat. No. 7,091,038, U.S. Published Patent Application Nos. 2002/0110861, 2004/0033600, 2004/0096942, 2005/0266457, and 2006/0100416, and International Published Application Nos. WO 01/70816, WO 02/066612, WO 02/066613, WO 02/066614, WO 02/066615, WO 02/29075, and WO 2005/108617, each of which is incorporated by reference in its entirety. An example of a non-steroidal ecdysone agonist-regulated system is the RheoSwitch® Mammalian Inducible Expression System (New England Biolabs, Ipswich, MA).

Still other promoter systems may include response elements including but not limited to a tetracycline (tet) response element (such as described by Gossen & Bujard (1992, Proc. Natl. Acad. Sci. USA 89:5547-551); or a hormone response element such as described by Lee et al. (1981, Nature 294:228-232); Hynes et al. (1981, Proc. Natl. Acad. Sci. USA 78:2038-2042); Klock et al. (1987, Nature 329:734-736); and Israel & Kaufman (1989, Nucl. Acids Res. 17:2589-2604) and other inducible promoters known in the art. Using such promoters, expression of the neutralizing antibody construct can be controlled, for example, by the Tet-on/off system (Gossen et al., 1995, Science 268:1766-9; Gossen et al., 1992, Proc. Natl. Acad. Sci. USA., 89(12):5547-51); the TetR-KRAB system (Urrutia R., 2003, Genome Biol., 4(10):231; Deuschle U et al., 1995, Mol Cell Biol. (4):1907-14); the mifepristone (RU486) regulatable system (Geneswitch; Wang Y et al., 1994, Proc. Natl. Acad. Sci. USA., 91(17):8180-4; Schillinger et al., 2005, Proc. Natl. Acad. Sci. U S A. 102(39):13789-94); the humanized tamoxifen-dep regulatable system (Roscilli et al., 2002, Mol. Ther. 6(5):653-63).

In another aspect of the invention, the gene switch is based on heterodimerization of FK506 binding protein (FKBP) with FKBP rapamycin associated protein (FRAP) and is regulated through rapamycin or its non-immunosuppressive analogs. Examples of such systems, include, without limitation, the ARGENT™ Transcriptional Technology (ARIAD Pharmaceuticals, Cambridge, Mass.) and the systems described in U.S. Pat. Nos. 6,015,709, 6,117,680, 6,479,653, 6,187,757, and 6,649,595, U.S. Publication No. 2002/0173474, U.S. Publication No. 200910100535, U.S. Patent No. 5,834,266, U.S. Patent No. 7,109,317, U.S. Patent No. 7,485,441, U.S.Patent No. 5,830,462, U.S. Patent No. 5,869,337, U.S. Patent No. 5,871,753, U.S. Patent No. 6,011,018, U.S. Patent No. 6,043,082, U.S. Patent No. 6,046,047, U.S. Patent No. 6,063,625, U.S. Patent No. 6,140,120, U.S. Patent No. 6,165,787, U.S. Patent No. 6,972,193, U.S. Patent No. 6,326,166, U.S. Patent No. 7,008,780, U.S. Patent No. 6,133,456, U.S. Patent No. 6,150,527, U.S. Patent No. 6,506,379, U.S. Patent No. 6,258,823, U.S. Patent No. 6,693,189, U.S. Patent No. 6,127,521, U.S. Patent No. 6,150,137, U.S. Patent No. 6,464,974, U.S. Patent No. 6,509,152, U.S. Patent No. 6,015,709, U.S. Patent No. 6,117,680, U.S. Patent No. 6,479,653, U.S. Patent No. 6,187,757, U.S. Patent No. 6,649,595, U.S. Patent No. 6,984,635, U.S. Patent No. 7,067,526, U.S. Patent No. 7,196,192, U.S. Patent No. 6,476,200, U.S. Patent No. 6,492,106, WO 94/18347, WO 96/20951, WO 96/06097, WO 97/31898, WO 96/41865, WO 98/02441, WO 95/33052, WO 99110508, WO 99110510, WO 99/36553, WO 99/41258,WO 01114387, ARGENT™ Regulated Transcription Retrovirus Kit, Version 2.0 (9109102), and ARGENT™ Regulated Transcription Plasmid Kit, Version 2.0 (9109/02), each of which is incorporated herein by reference in its entirety. The Ariad system is designed to be induced by rapamycin and analogs thereof referred to as "rapalogs". Examples of suitable rapamycins are provided in the documents listed above in connection with the description of the ARGENT™ system. In one embodiment, the molecule is rapamycin [e.g., marketed as Rapamune™ by Pfizer]. In another embodiment, a rapalog known as AP21967 [ARIAD] is used. Examples of these dimerizer molecules that can be used in the present invention include, but are not limited to rapamycin, FK506, FK1012 (a homodimer of FK506), rapamycin analogs ("rapalogs") which are readily prepared by chemical modifications of the natural product to add a "bump" that reduces or eliminates affinity for endogenous FKBP and/or FRAP. Examples of rapalogs include, but are not limited to such as AP26113 (Ariad), AP1510 (Amara, J.F., et al.,1997, Proc Natl Acad Sci USA, 94(20): 10618-23) AP22660, AP22594, AP21370, AP22594, AP23054, AP1855, AP1856, AP1701, AP1861, AP1692 and AP1889, with designed 'bumps' that minimize interactions with endogenous FKBP. Still other rapalogs may be selected, *e.g.*, AP23573 [Merck].

Methods for generating and isolating AAVs suitable for use as vectors are known in the art. *See* generally, *e.g.,* Grieger & Samulski, 2005, "Adeno-associated virus as a gene therapy vector: Vector development, production and clinical applications," Adv. Biochem. Engin/Biotechnol. 99: 119-145; Buning et a/., 2008, "Recent developments in adeno-associated virus vector technology," J. Gene Med. 10:717-733; and the references cited below, each of which is incorporated herein by reference in its entirety.

For packaging a transgene into virions, the ITRs are the only AAV components required in *cis* in the same construct as the transgene. The cap and rep genes can be supplied in *trans.* Accordingly, DNA constructs can be designed so that the AAV ITRs flank the coding sequence for the anti-pathogen construct (or subunits thereof, or subunits thereof fused to a dimerizable domain which is part of a regulatable promoter), thus defining the region to be amplified and packaged - the only design constraint being the upper limit of the size of the DNA to be packaged (approximately 4.5 kb). Adeno-associated virus engineering and design choices that can be used to save space are described below.

An AAV viral vector may include using multiple transgenes. In certain situations, a different transgene may be used to encode each subunit of a protein (e.g., an immunoglobulin heavy chain, an immunoglobulin light chain), or to encode different peptides or proteins (e.g., of the anti-pathogen construct, or a transcription factor, or another protein). This is desirable when the size of the DNA encoding the protein subunit is large, *e.g.*, for a full-length immunoglobulin. In one embodiment, a cell produces the multi-subunit protein following infected/transfection with the virus containing each of the different subunits. In another embodiment, different subunits of a protein may be encoded by the same transgene. In this case, a single transgene includes the DNA encoding each of the subunits, with the DNA for each subunit separated by an internal ribozyme entry site (IRES) or a self-cleaving peptide (*e.g.*, 2A). An IRES is desirable when the size of the DNA encoding each of the subunits is small, *e.g.,* the total size of the DNA encoding the subunits and the IRES is less than five kilobases. As an alternative to an IRES, the DNA may be separated by sequences encoding a 2A peptide, which self-cleaves in a post-translational event. See, *e.g.*, ML Donnelly, et al, (Jan 1997) J. Gen. Virol., 78(Pt 1): 13-21; S. Furler, S et al, (June 2001) Gene Ther., 8(11):864-873; H. Klump, et al., (May 2001) Gene Ther., 8(10):811-817. This 2A peptide is significantly smaller than IRES, making it well suited for use when space is a limiting factor. More often, when the transgene is large, consists of multi-subunits, or two transgenes are co-delivered, rAAV carrying the desired transgene(s) or subunits are co-administered to allow them to concatamerize *in vivo* to form a single vector genome. In such an embodiment, a first AAV may carry an expression cassette which expresses a single transgene and a second AAV may carry an expression cassette which expresses a different transgene for co-expression in the host cell. However, the selected transgene may encode any biologically active product or other product, *e.g.*, a product desirable for study.

In addition to the elements identified above for the expression cassette, the vector also includes conventional control elements which are operably linked to the coding sequence in a manner which permits transcription, translation and/or expression of the encoded product (*e.g.*, a neutralizing antibody or a portion thereof) in a cell transfected with the plasmid vector or infected with the virus produced by the invention. As used herein, "operably linked" sequences include both expression control sequences that are contiguous with the gene of interest and expression control sequences that act in *trans* or at a distance to control the gene of interest.

Expression control sequences include appropriate transcription initiation, termination, promoter and enhancer sequences; efficient RNA processing signals such as splicing and polyadenylation (polyA) signals; sequences that stabilize cytoplasmic mRNA; sequences that enhance translation efficiency (*i.e.*, Kozak consensus sequence); sequences that enhance protein stability; and when desired, sequences that enhance secretion of the encoded product.

For use in producing an AAV viral vector (*e.g.*, a recombinant (r) AAV), the expression cassette can be carried on any suitable vector, *e.g.*, a plasmid, which is delivered to a packaging host cell. The plasmids useful in this invention may be engineered such that they are suitable for replication and packaging in prokaryotic cells, mammalian cells, or both. Suitable transfection techniques and packaging host cells are known and/or can be readily designed by one of skill in the art.

Methods of preparing AAV-based vectors (*e.g*., having an AAV9 or another AAV capsid) are known. *See, e.g.*, US Published Patent Application No. 2007/0036760 (February 15, 2007), which is incorporated by reference herein. The invention is not limited to the use of AAV9 or other clade F AAV amino acid sequences, but encompasses peptides and/or proteins containing the terminal β-galactose binding generated by other methods known in the art, including, *e.g.*, by chemical synthesis, by other synthetic techniques, or by other methods. The sequences of any of the AAV capsids provided herein can be readily generated using a variety of techniques. Suitable production techniques are well known to those of skill in the art. See, *e.g.*, Sambrook et al, Molecular Cloning: A Laboratory Manual, Cold Spring Harbor Press (Cold Spring Harbor, NY). Alternatively, peptides can also be synthesized by the well-known solid phase peptide synthesis methods (Merrifield, (1962) J. Am. Chem. Soc., 85:2149; Stewart and Young, Solid Phase Peptide Synthesis (Freeman, San Francisco, 1969) pp. 27-62). These methods may involve, e.g., culturing a host cell which contains a nucleic acid sequence encoding an AAV capsid; a functional rep gene; a minigene composed of, at a minimum, AAV inverted terminal repeats (ITRs) and a transgene; and sufficient helper functions to permit packaging of the minigene into the AAV capsid protein. These and other suitable production methods are within the knowledge of those of skill in the art and are not a limitation of the present invention.

The components required to be cultured in the host cell to package an AAV minigene in an AAV capsid may be provided to the host cell in *trans.* Alternatively, any one or more of the required components (*e.g.*, minigene, *rep* sequences, *cap* sequences, and/or helper functions) may be provided by a stable host cell which has been engineered to contain one or more of the required components using methods known to those of skill in the art. Most suitably, such a stable host cell will contain the required component(s) under the control of an inducible promoter. However, the required component(s) may be under the control of a constitutive promoter. Examples of suitable inducible and constitutive promoters are provided herein, in the discussion of regulatory elements suitable for use with the transgene. In still another alternative, a selected stable host cell may contain selected component(s) under the control of a constitutive promoter and other selected component(s) under the control of one or more inducible promoters. For example, a stable host cell may be generated which is derived from 293 cells (which contain E1 helper functions under the control of a constitutive promoter), but which contains the rep and/or cap proteins under the control of inducible promoters. Still other stable host cells may be generated by one of skill in the art.

The minigene, *rep* sequences, *cap* sequences, and helper functions required for producing the rAAV of the invention may be delivered to the packaging host cell in the form of any genetic element which transfer the sequences carried thereon. The selected genetic element may be delivered by any suitable method, including those described herein. The methods used to construct any embodiment of this invention are known to those with skill in nucleic acid manipulation and include genetic engineering, recombinant engineering, and synthetic techniques. See, *e.g.*, Sambrook et al, Molecular Cloning: A Laboratory Manual, Cold Spring Harbor Press, Cold Spring Harbor, NY. Similarly, methods of generating rAAV virions are well known and the selection of a suitable method is not a limitation on the present invention. See, *e.g.*, K. Fisher et al, (1993) J. Virol., 70:520-532 and US Patent No. 5,478,745.

Unless otherwise specified, the AAV ITRs, and other selected AAV components described herein, may be readily selected from among any AAV. Further, more than one AAV source may provide elements to an AAV vector. For example, as described above, a pseudotyped AAV may contain ITRs from a source which differs from the source of the AAV capsid. Additionally or alternatively, a chimeric AAV capsid may be utilized. Still other AAV components may be selected. Sources of such AAV sequences are described herein and may also be isolated or obtained from academic, commercial, or public sources (*e.g*., the American Type Culture Collection, Manassas, VA). Alternatively, the AAV sequences may be obtained through synthetic or other suitable means by reference to published sequences such as are available in the literature or in databases such as, *e.g.*, GenBank®, PubMed®, or the like.

The AAV vectors may be suspended in a physiologically compatible carrier for administration to a human or non-human mammalian patient. Suitable carriers may be readily selected by one of skill in the art in view of the route of delivery. For example, one suitable carrier includes saline, which may be formulated with a variety of buffering solutions (*e.g.*, phosphate buffered saline). Other exemplary carriers include sterile saline, lactose, sucrose, calcium phosphate, gelatin, dextran, agar, pectin, peanut oil, sesame oil, and water. The selection of the carrier is not a limitation of the present invention. Optionally, the compositions of the invention may contain, in addition to the rAAV and carrier(s), other conventional pharmaceutical ingredients, such as preservatives, or chemical stabilizers. Suitable exemplary preservatives include chlorobutanol, potassium sorbate, sorbic acid, sulfur dioxide, propyl gallate, the parabens, ethyl vanillin, glycerin, phenol, and parachlorophenol. Suitable chemical stabilizers include gelatin and albumin.

The selection of the carrier or other excipients for preparation of the AAV composition(s) is not a limitation of the present invention. One of skill in the art may select other suitable carriers, including those particularly well adapted for intranasal delivery.

### Regimens

Suitably, the compositions described herein are designed to carry at least one type of AAV viral vector carrying at least one anti-pathogen construct. The compositions may contain two or more different AAV viral vectors. Such different AAV viral vectors may contain different subunits of the same anti-pathogen construct, different anti-pathogenic constructs, and/or the same anti-pathogenic constructs in AAV viral vectors which differ in one or more element, *e.g.*, capsid, promoter, enhancer, polyA, marker gene, etc. In one embodiment, the anti-pathogen construct neutralizes more than one subtype of said pathogen.

The present invention is well suited for rapid response following the emergence of an unpredictable strain of a pathogen (*e.g.*, against a pandemic virus), as it permits generation of an anti-pathogen construct as a neutralizing antibody is available.

In one embodiment, the invention provides for a passive immunization regimen in which a combination of AAV vectors which comprise different anti-pathogen constructs are delivered to the subject. In one embodiment, a single composition contains more than one different type of anti-pathogen construct.

The anti-pathogen construct is selected based on the causative agent (pathogen) for the disease against which protection is sought. These pathogens may be of viral, bacterial, or fungal origin, and may be used to prevent infection in humans against human disease, or in non-human mammals or other animals to prevent veterinary disease.

Examples of such viruses include influenza virus from the orthomyxovirudae family, which includes: Influenza A, Influenza B, and Influenza C. The type A viruses are the most virulent human pathogens. The serotypes of influenza A which have been associated with pandemics include, H1N1, which caused Spanish Flu in 1918, and Swine Flu in 2009; H2N2, which caused Asian Flu in 1957; H3N2, which caused Hong Kong Flu in 1968; H5N1, which caused Bird Flu in 2004; H7N7; H1N2; H9N2; H7N2; H7N3; and H10N7.

Broadly neutralizing antibodies against influenza A have been described. As used herein, a "broadly neutralizing antibody" refers to a neutralizing antibody which can neutralize multiple strains from multiple subtypes. For example, CR6261 [The Scripps Institute/ Crucell] has been described as a monoclonal antibody that binds to a broad range of the influenza virus including the 1918 "Spanish flu" (SC1918/H1) and to a virus of the H5N1 class of avian influenza that jumped from chickens to a human in Vietnam in 2004 (Viet04/H5). CR6261 recognizes a highly conserved helical region in the membrane-proximal stem of hemagglutinin, the predominant protein on the surface of the influenza virus. This antibody is described in WO 2010/130636, incorporated by reference herein. Another neutralizing antibody, F10 [XOMA Ltd] has been described as being useful against H1N1 and H5N1. [Sui et al, Nature Structural and Molecular Biology (Sui, et al. 2009, 16(3):265-73)] Other antibodies against influenza, *e.g.*, Fab28 and Fab49, may be selected. See, e.g., WO 2010/140114 and WO 2009/115972, which are incorporated by reference. Still other antibodies, such as those described in WO 2010/010466, US Published Patent Publication US/2011/076265, and WO 2008/156763, may be readily selected.

Other target pathogenic viruses include, arenaviruses (including funin, machupo, and Lassa), filoviruses (including Marburg and Ebola), hantaviruses, picornoviridae (including rhinoviruses, echovirus), coronaviruses, paramyxovirus, morbillivirus, respiratory synctial virus, togavirus, coxsackievirus, parvovirus B19, parainfluenza, adenoviruses, reoviruses, variola (Variola major (Smallpox)) and Vaccinia (Cowpox) from the poxvirus family, and varicella-zoster (pseudorabies).

Viral hemorrhagic fevers are caused by members of the arenavirus family (Lassa fever) (which family is also associated with Lymphocytic choriomeningitis (LCM)), filovirus (ebola virus), and hantavirus (puremala). The members of picornavirus (a subfamily of rhinoviruses), are associated with the common cold in humans. The coronavirus family, which includes a number of non-human viruses such as infectious bronchitis virus (poultry), porcine transmissible gastroenteric virus (pig), porcine hemagglutinatin encephalomyelitis virus (pig), feline infectious peritonitis virus (cat), feline enteric coronavirus (cat), canine coronavirus (dog). The human respiratory coronaviruses, have been putatively associated with the common cold, non-A, B or C hepatitis, and sudden acute respiratory syndrome (SARS). The paramyxovirus family includes parainfluenza Virus Type 1, parainfluenza Virus Type 3, bovine parainfluenza Virus Type 3, rubulavirus (mumps virus, parainfluenza Virus Type 2, parainfluenza virus Type 4, Newcastle disease virus (chickens), rinderpest, morbillivirus, which includes measles and canine distemper, and pneumovirus, which includes respiratory syncytial virus (RSV). The parvovirus family includes feline parvovirus (feline enteritis), feline panleucopeniavirus, canine parvovirus, and porcine parvovirus. The adenovirus family includes viruses (EX, AD7, ARD, O.B.) which cause respiratory disease.

A neutralizing antibody construct against a bacterial pathogen may also be selected for use in the present invention. In one embodiment, the neutralizing antibody construct is directed against the bacteria itself. In another embodiment, the neutralizing antibody construct is directed against a toxin produced by the bacteria. Examples of airborne bacterial pathogens include, *e.g., Neisseria meningitidis* (meningitis), *Klebsiella pneumonia* (pneumonia), *Pseudomonas aeruginosa* (pneumonia), *Pseudomonas pseudomallei* (pneumonia), *Pseudomonas mallei* (pneumonia), Acinetobacter (pneumonia), *Moraxella catarrhalis, Moraxella lacunata, Alkaligenes, Cardiobacterium, Haemophilus influenzae* (flu), *Haemophilus parainfluenzae, Bordetella pertussis* (whooping cough), *Francisella tularensis* (pneumonia/fever), *Legionella pneumonia* (Legionnaires disease), *Chlamydia psittaci* (pneumonia), *Chlamydia pneumoniae (*pneumonia), *Mycobacterium tuberculosis* (tuberculosis (TB)), *Mycobacterium kansasii (*TB*),*
*Mycobacterium avium* (pneumonia), *Nocardia asteroides* (pneumonia), *Bacillus anthracis* (anthrax), *Staphylococcus aureus* (pneumonia), *Streptococcus pyogenes* (scarlet fever), *Streptococcus pneumoniae* (pneumonia), *Corynebacteria diphtheria* (diphtheria), *Mycoplasma pneumoniae* (pneumonia).

The causative agent of anthrax is a toxin produced by *Bacillius anthracis.* Neutralizing antibodies against protective agent (PA), one of the three peptides which form the toxoid, have been described. The other two polypeptides consist of lethal factor (LF) and edema factor (EF). Anti-PA neutralizing antibodies have been described as being effective in passively immunization against anthrax. *See, e.g.*, US Patent number 7,442,373; R. Sawada-Hirai et al, J Immune Based Ther Vaccines. 2004; 2: 5. (on-line 2004 May 12). Still other anti-anthrax toxin neutralizing antibodies have been described and/or may be generated. Similarly, neutralizing antibodies against other bacteria and/or bacterial toxins may be used to generate an AAV-delivered anti-pathogen construct as described herein.

Other infectious diseases may be caused by airborne fungi including, *e.g., Aspergillus* species, *Absidia corymbifera*, *Rhixpus stolonifer*, *Mucor plumbeaus*, *Cryptococcus neoformans*, *Histoplasm capsulatum, Blastomyces dermatitidis, Coccidioides immitis, Penicillium* species, *Micropolysporafaeni*, *Thermoactinomyces vulgaris*, *Alternaria alternate*, *Cladosporium* species, *Helminthosporium*, and *Stachybotrys* species.

In addition to airborne infectious disease conditions which affect humans, many of which are described above, passive immunization according to the invention may be used to prevent conditions associated with direct inoculation of the nasal passages, e.g., conditions which may be transmitted by direct contact of the fingers with the nasal passages. These conditions may include fungal infections (*e.g.,* athlete's foot), ringworm, or viruses, bacteria, parasites, fungi, and other pathogens which can be transmitted by direct contact. In addition, a variety of conditions which affect household pets, cattle and other livestock, and other animals. For example, in dogs, infection of the upper respiratory tract by canine sinonasal aspergillosis causes significant disease. In cats, upper respiratory disease or feline respiratory disease complex originating in the nose causes morbidity and mortality if left untreated. Cattle are prone to infections by the infectious bovine rhinotracheitis (commonly called IBR or red nose) is an acute, contagious virus disease of cattle. In addition, cattle are prone to Bovine Respiratory Syncytial Virus (BRSV) which causes mild to severe respiratory disease and can impair resistance to other diseases. Still other pathogens and diseases will be apparent to one of skill in the art.

An antibody, and particularly, a neutralizing antibody, against a pathogen such as those exemplified herein, may be used to generate an anti-pathogen construct. Monoclonal antibodies (mAbs) with broad neutralizing capacity can be identified using antibody phage display to screen libraries from donors recently vaccinated with the seasonal flu vaccine, from non-immune humans or from survivors of a natural infection. In the case of influenza, antibodies have been identified which neutralize more than one influenza subtype by blocking viral fusion with the host cell. This technique may be utilized with other infections to obtain a neutralizing monoclonal antibody. *See, e.g.,* US 5,811,524, which describes generation of anti-respiratory syncytial virus (RSV) neutralizing antibodies. The techniques described therein are applicable to other pathogens. Such an antibody may be used intact or its sequences (scaffold) modified to generate an artificial or recombinant neutralizing antibody construct. Such methods have been described [see, e.g., WO 2010/13036; WO 2009/115972; WO 2010/140114].

In another embodiment, other diseases, including genetic, acquired or infectious diseases may be treated using the regimen and compositions of the invention.

In another embodiment, an artificial or recombinant neutralizing antibody construct may be generated from monoclonal antibodies prepared using hybridoma methods, such as those described by Kohler and Milstein (Nature, 1975, 256:495). In one embodiment, mouse, rat, hamster or other host animals, is immunized with an immunizing agent to generate lymphocytes that produce antibodies with binding specificity to the immunizing antigen. In an alternative approach, the lymphocytes may be immunized in vitro. Human antibodies can be produced using techniques such as phage display libraries (Hoogenboom and Winter, J. Mol. Biol, 1991, 227:381, Marks et al., J. Mol. Biol. 1991, 222:581).

In one embodiment, an anti-pathogen construct is from a species which differs from the species of the subject to which it is administered. In other words, an equine antibody is administered to a human, or a rat antibody is delivered to cattle. In still other embodiments, the anti-pathogen construct is a chimeric in which the scaffold of the antibody is engineered to contain more species-specific sequences. For example, methods for humanizing non-human antibodies are well known. Humanization can be performed following the method of Winter et al. (Jones et al., Nature, 1986, 321:522; Riechmann et al., Nature, 1988, 332:323; Verhoeyen et al., Science, 1988, 239:1534) by substituting rodent CDR sequences or CDRs for the corresponding sequences of a human antibody. Such humanized antibodies are chimeric antibodies (U.S. Pat. No. 4,816,567). Typically, humanized antibodies are antibodies where CDR residues are substituted by residues from analogous sites in rodent antibodies. Antibodies of the invention may be single-chain variable fragment antibody (scFV). Recombinant approaches have led to the development of single chain variable fragment antibody (scFv). A monomeric scFv has a molecular mass of only about 30 kDa, which is expressed in a variety of systems as a single VL-VH pair linked by a Gly/Ser-rich synthetic linker (Berezov A. et al., 2001, J Med Chem 44:2565). When expressed in bacteria or eukaryotic cells, the scFv folds into a conformation similar to the corresponding region of the parental antibody. It was shown to retain comparable affinity to that of a Fab (Kortt et al., 1994, Eur J Biochem 221:151). ScFvs are amenable to various genetic modifications such as humanization and the production of fusion proteins to enhance their potential as therapeutic agents.

Anti-pathogen constructs such as these neutralizing antibody constructs are engineered into expression cassettes and AAV vectors using techniques described herein and known to those of skill in art.

In one embodiment, a composition of the invention may include one or more AAV which contain elements necessary for the inducible/regulatable promoter. For example, an AAV carrying a neutralizing antibody construct may be co-administered with a different AAV carrying a transcription factor which forms a part of the regulatable expression system. Examples of such systems include those described, e.g., in International Patent Application No. PCT/US11/20213, filed March 28, 2011, which is incorporated by reference herein. In such an embodiment, an anti-pathogen construct or a portion thereof (*e.g.*, a light chain, heavy chain, or another fragment) may be expressed as a fusion protein. Such fusion proteins combine to form an "anti-pathogen construct" as defined herein following activation with a transcription factor or induction by a pharmacologic agent.

Thus, the compositions may carry a single type of AAV viral vector carrying at least one anti-pathogen construct. Alternatively, two or more AAV viral vectors may be co-administered. A composition may carry two or more AAV viral vectors which combine in vivo to form a single anti-pathogen construct. Thus, a composition may be delivered which contains two or more different AAV viral vectors. Such different AAV viral vectors may contain different subunits of the same anti-pathogen construct, different anti-pathogenic constructs, and/or the same anti-pathogenic constructs in AAV viral vectors which differ in one or more element, *e.g.*, capsid, promoter, enhancer, polyA, marker gene, etc, or one of the AAV viral vector may contain another transgene desired to be co-expressed with the anti-pathogen construct. In one embodiment, the anti-pathogen construct is neutralizes more than one strain and/or more than one subtype of said pathogen.

In another embodiment, the invention permits delivery of a nucleic acid construct to the nasopharynx which can be used to "knock-out" or "knock-down" a gene, *e.g.*, for treatment of diseases associated with the nasopharyngeal region, including, *e.g.*, nasopharyngeal cancers (NPC), sinusitis, tonsillitis, allergic rhinitis, *etc.* or example, RNA molecules which are directed against Epstein-barr virus (which is associated with nasopharyngeal cancer) may be selected for delivery to the nasopharyngeal cells. These include, *e.g.*, micro RNAs (miRNAs), including BamHI-A region (BART) miRNAs, and small interfering RNAs (siRNA), may be engineered into an AAV vector for delivery to the nasopharnyx. In another embodiment, a RNA or antibody construct, *e.g.,* an anti-VEGF antibody (such as bevacizumab) may be selected, or RNA or antibody directed against another angiogenesis protein, may be engineered into an AAV as described herein. In still another embodiment, an AAV vector with an RNA (e.g., shRNA or siRNA) against NF-kappaB is delivered to the site of an NPC to suppress or knockout expression thereof, resulting in the up-regulation of E-cadherin. In still a further embodiment, AAV vectors are used to suppress or knockout expression of NF-kappaB to up-regulate E-cadherin expression and minimize spread of NPC.

In yet another embodiment, the invention may be used as a tool in animal models to allow for identification of novel therapeutic targets for diseases associated with the nasopharynx.

Dosages of the viral vector will depend primarily on factors such as the condition being treated, the age, weight and health of the patient, and may thus vary among patients. For example, a therapeutically effective human dosage of the viral vector is generally in the range of about 1 x 10⁹ to about 1 x 10¹⁶ genomes AAV vector, or about 1 x 10¹⁰ to about 10¹⁵ or about 1 x 10¹⁰ to about 10¹⁴, or about 10¹³, or about 10¹², delivered by a route or formulation or intranasally as a liquid at a volume which substantially avoids delivery to and transduction of lung cells. In one embodiment, the delivery of low volume AAV restricts more than about 70%, more than 80%, more than 90%, more than about 95%, or more than about 99% of expression to the nasal epithelium where a constitutive promoter is utilized. Use of a tissue-specific, cell specific, or an inducible or regulatable promoter delivered locally to the nose further limits expression to the nasal/nasopharynx epithelium.

More particularly, it has been described in the literature that instillation volume influences the distribution of viral vectors and that a relatively high volume is required to provide a therapeutically effective amount of a transgene to the human lung. In the present invention, if the viral vectors are delivered as an intranasal spray (*e.g.*, an aerosol, etc), it is delivered to the subject at a relatively low instillation volume in order to minimize lung transduction. In another embodiment, the viral vectors may be delivered intranasally through a means other than an intranasal spray, *e.g.*, injection. In another embodiment, the compositions carrying the vectors are delivered sequentially to each nostril/nare through a bolus containing the viral vectors.

Such a dose for a human adult may be in the range of about 0.1 mL to about 50 mL, or about 0.1 mL to about 50 mL, about 1 mL to about 40 mL, about 5 mL to about 35 mL, or about 0.1 mL, about 1 mL, about 5 mL, about 10 mL, about 15 mL, about 20 mL, about 25 mL, about 30 mL, about 35 mL, about 40 mL, about 45 mL, about 50 mL, about 55 mL, about 60 mL, about 65 mL, about 70 mL. A preferred human dosage may be about 5 x 10¹⁰ to 5 x 10¹³ AAV genomes per 1 kg. The dosage for larger mammals may be adjusted as needed (*e.g.*, increased for bovine, and horses) or decreased for smaller mammals (e.g., sheep, goats).

In one embodiment, useful levels of expression of the anti-pathogen construct are detectable in the nasopharynx cells of said subject within about 3 days to about 7 days following administration]. Where expression is controlled by an inducible promoter, the inducer may be delivered to the subject within this time period, i.e., after about 3 days following administration of the AAV vectors. However, in certain embodiments, delivery may be earlier and later following AAV delivery. In one embodiment, the AAV viral vector(s) transduces the subject's nasopharynx cells in the presence of high level serum-circulating AAV neutralizing antibodies.

In one embodiment, a regulatable promoter permits further control of the expression of the anti-pathogen construct(s), through controlling contacting of the cells carrying the AAV carrying the coding sequences for the anti-pathogen construct(s). More particularly, the ligand for the regulatable promoter (*e.g.*, rapamycin or a rapalog) may be delivered locally to the AAV-transfected cells of the nasopharynx. This local delivery may be by intranasal injection. However, in another embodiment, the inventors have found that the rapamycin or rapalog is capable of regulating expression of the anti-pathogen construct if delivered topically to the cells. Topical delivery may be by delivering a bolus containing the rapamycin or rapalog to each nostril/nare. In another embodiment, topical delivery may be by formulating the rapamycin or rapalog in a suitable composition for topical delivery (*e.g.*, a cream or gel). In still another embodiment, the compositions and methods may be adapted for use with another ligand for anti-pathogen constructs which are under the control of a different regulatable system.

In one embodiment, gene expression is controlled in a dose dependent manner by the regulating pharmacologic compound. In other words, the level of gene expression is lower when low levels of the compound are delivered and increased by increasing the amount of compound. For example, a rapalog (*e.g.*, AP21967) may be administered at a dose of from about 0.1 to about 100 nM, or adjusted as needed or desired. These compositions have been designed to be delivered systemically, *e.g.*, by oral medication or intravenously.

Inducing agents (e.g., rapamycin or a rapalog) have been described as being delivered systemically, *e.g.*, by oral or intraperitoneal administration, *e.g.*, by injection. However, the present inventors have found that it is possible to induce expression in nasal cells following local administration of the inducer. Such local administration may involve intranasal injection. However, in another embodiment, this local administration involves topical administration. Such topical administration may be performed through use of a bolus delivery to each nostril/nare of a subject. A liquid suspension or solution containing the inducing agent (e.g., rapamycin or a rapalog) may be delivered topically, *e.g.,* by blocking and instilling each nostril (nare) and allowing the liquid to remain in the nostril for a period of time and then repeating the procedure in the other nostril. Alternatively, the compound may be formulated for delivery as a gel, cream, or other composition which can be applied to the nostril(s)/nare(s). Suitably, the volume of the liquid delivered is controlled such that there is an insufficient amount to reach the lung. For example, a rapalog (*e.g.*, AP21967) may be administered at a dose of about 0.1 to about 100 nM, or about 0.5 to 1 mg, or adjusted as needed or desired.

In one embodiment, the inducing agent (*e.g.*, a pharmacologic compound such as rapamycin or a rapalog) is delivered to the subject between 5 days to 12 weeks, or longer, following delivery of the AAV composition to the cell. Optionally, the inducing agent is dosed periodically in order to provide for short-term expression (*e.g.*, 3 to 7, or about 5 days) of the anti-pathogen agent. In one embodiment, prophylactively effective levels of expression of the anti-pathogen construct is detectable in the nasopharynx of said subject within about twenty-four hours following delivery of an adequate dose of the inducing compound. However, expression levels may in certain cases be detectable as quickly as about 8 hours, about 12 hours, or about 18 hours following induction. In certain cases, expression may be deferred, e.g., through administration of a delayed release formulation containing the inducing agent. The inducing agent may be delivered once per week for any of weeks 1 to 12 following delivery of the AAV compositions, optionally with breaks of 7 days (one week) or more between inductions. Optionally, the amount of inducing agent may change in subsequent inductions. For example, it may be desirable to start with a high dose of the pharmacologic compound and then use lower doses for subsequent inductions. Alternatively, it may be desirable to use a higher dose of the pharmacologic agent when the induction is performed at a time more remote to the delivery of the AAV (e.g., a higher amount of inducing agent may be desired after more than 8, 10, or 12 weeks has passed since delivery of the AAV(s) carrying the sequence encoding the anti-pathogen compound(s).

The following examples illustrate several aspects and embodiments of the invention.

### Example 1 - Construction of AAV - Ab vectors.

Specific human broadly neutralizing antibodies are cloned in highly efficient lung-directed AAV vectors. Initially, CR6261 [D.C. Ekiert, et al, "Antibody recognition of a highly conserved influenza virus epitope", Science, 324 (5924), 246-251 (2009)]a broadly-neutralizing Ab isolated by Crucell (Holland), is cloned into an AAV vector construct and produce AAV9 vector. The sequences of this antibody are available from WO 2010/130636-A1 (18-NOV-2010), *see*, *e.g.* sequences 186 and 184), and the NCBI data base accession numbers: 3GBN_L GI: 224983685 (light chain), 3GBN_H (heavy chain). Briefly, to generate a mAb AAV expression construct, VH and VL [lambda] domains from CR6261 are cloned into constitutive expressing AAV vectors. This particular IgG1 constant region is known to support proper pairing with lambda light chains and to confer effector functions that support virus neutralization. Protein expression levels from the Ab will be confirmed *in vitro* by Western Blot and ELISA using a polyclonal anti-human IgG Ab.

### Example 2 - Protection of animal models following challenge with pathogenic viruses.

### A. Mouse models

In initial experiments the mAb vectors will be used. Briefly, the efficacy of the AAV-MAb vector will be assessed in BALB/c mice by delivering the AAV vector intranasally (IN). The titer of Ab will be assessed in nasal lavage and bronchoalveolar lavage fluids as well as in serum. Twenty-eight days later the vector-treated mice will be challenged under ABSL2 conditions using an IN bolus of a lethal dose of the mouse-adapted A/Puerto Rico/8/34 (H1N1) flu strain (PR8-MTS). Mice will be monitored daily for clinical signs of influenza infection apparent as interstitial pneumonia and significant loss (<30%) of body weight. These challenge experiments will be repeated with a lethal dose of ABSL3+ level pathogenic strains of influenza H5N1 [Hanoi 2005: A/Hanoi/30408/2005; Vietnam 2004: A/Vietnam/1203/2004; Hong Kong 1997: A/Hong Kong/483/1997; Indonesia 2005 A/Indonesia/05/2005] and H1N1 [1918: A/South Carolina/1/18].

Influenza transmission by the action of a sneeze or a cough will be modelled. Mice treated with the passive vaccine will be challenged with aerosolized A/PR/8/34 (H1N1) flu strain under ABSL2 conditions at the University of Pennsylvania, using the middlebrook airborne infection apparatus. Similar experiments will be conducted under ABSL3+ conditions using the highly pathogenic H5N1 and H1N1 strains mentioned above.

These experiments will also be performed with the pharmacologically-regulated AAV vector to evaluate the therapeutic potential of a prophylactic vaccine, the expression of which will be regulated by rapamycin. Pilot data in mice demonstrate that the onset of gene expression is fast and peaks within 24 hrs at levels similar to those conferred by the constitutive-expressing vector. The additional advantage of a pharmacologically-regulated AAV vector is its improved safety. The Ab would only be expressed for a few days at a time period that is critical for the severity and spread of the infection, and Ab expression wanes with time to return to background levels within 4-5 days, thus minimizing the risk of immune complex formation, a likely consequence of continuous Ab expression.

While mouse models can provide important information regarding the level and duration of expression of the passive vaccine, the predictive value of mouse studies in translational studies remains debatable. As such, the AAV-Ab constructs found to protect mice upon challenge with the highly pathogenic strains of influenza will be evaluated in the well-characterized ferret model.

### B. Ferret models:

The ferret is considered to more closely model the clinical sequelae of influenza infection in humans, and has been shown to predict the value of prophylactic treatments. For these experiments, groups of ferrets will be immunized with the constitutive or pharmacologically-regulated AAV-Ab vector injected intramuscularly (IM) or delivered intranasally (IN). At day 28, nasal washes and serum will be collected to assess the level of Ab on the mucosal surface and in the circulation, respectively. Ferrets will then be challenged with a lethal dose of the pathogenic strains of influenza, delivered as liquid or as aerosol. Clinical signs of sneezing, inappetence, dyspnea and level of activity will be assessed daily. Ferrets that exhibit behavioral signs of distress will be euthanized.

### Example 3 - Protection of animal models following challenge with SARS-CoV and EBOV

The therapeutic potential the passive vaccine of the invention is assessed in challenge studies with EBOV in mice and guinea pigs, and SARS-CoV in ferrets. In a similar manner as described above for influenza, mice and guinea pigs will be inoculated IN with AAV-expressing neutralizing anti-EBOV Abs (anti-ebola antibodies) and challenged with the mouse-adapted Zaire EBOV (EBO-Z) virus (mice) and the EBO-Z virus (guinea pigs). For the SARS-CoV challenge studies, ferrets will be inoculated IN with AAV-expressing anti-SARS-CoV Ab and challenged with SARS-CoV of the Toronto-2 strain. As mentioned earlier, mice and ferrets will also be subjected to an aerosol exposure of EBO-Z virus and SARS-CoV to model the infection following exposure to a sneeze or a cough.

### Example 4 - Topical Ramamycin Induces Expression from Nasal Epithelial Cells Transduced with AAV Vectors

The utility of inducible vectors in the nasal airway adds another level of safety, as expression of the transgene product is regulated *via* the administration of rapamycin or rapalog, which can have immunosuppressive effects when administered systemically. The following study assessed the topical, as opposed to the systemic, administration of rapamycin to activate AAV-mediated gene expression.

### A. Vector Injections

C57BL/6 mice (6 to 8 weeks of age) were purchased from Charles River Laboratories (Wilmington, MA) and kept under pathogen-free conditions at the Animal Facility of the Translational Research Laboratories. Mice were anesthetized using an intraperitoneal injection of ketamine/xylazine. For vector administrations, mice were inoculated intranasally (IN) with 15µl in the right and left nostril for a total dose of 10¹¹ genome copies (GC) in 30µl. All animal procedures were approved by the Institutional Animal Care and Use Committees of the University of Pennsylvania.

### B. Imaging

Mice were anaesthetized with ketamine/xylazine and ∼5 mins later 15µl of 15 mg/ml D-luciferin (Caliper, USA) was delivered to the right and left nostril IN. Five mins later mice were imaged using the IVIS Xenogen imaging system. Quantitation of signal was calculated using the Living Image® 3.0 Software.

### C. Results

Groups of mice were injected with a) the AAV9 inducible vector expressing luciferase (luc) and the AAV9 vector expressing the transcription factor (Tf) or b) the constitutive AAV2/9 vector expressing luc. As controls, mice were injected with (c) the inducible AAV9 vector expressing luc, (d) the AAV9 vector expressing Tf or (e) PBS (naïve mice).

Mice were injected intraperitoneally with rapamycin (1 mg/kg) or intranasally with 1 mg/kg rapamycin (delivered as 5 µl in the right and left nostrils) to induce gene expression and imaged. The first induction with rapamycin (ip, 1 mg/kg) was at day 17 following AAV delivery, the second induction with rapamycin (ip, 1mg/kg), the third induction with rapamycin was at day 82 (1 mg/kg, ip); the fourth induction with rapamycin (ip, 1 mg/kg) was at day 109, and the fifth inducation with rapamycin was at day 123 (intranasal, 0.5 - 1 mg/kg). Background luminescence was set to 1000 photons/sec (p/s) based on measurements of luc expression of the control groups. 24 hr after each induction (Figure 1), the level of luc expression achieved was similar to that conferred by the constitutive AAV9 vector (as shown in Figure 3). Four rapamycin inductions were performed 3-6 weeks apart and levels of 100-1000-fold induction were demonstrated for each mouse (n=4/group) (Figure 1). In addition, topical instillation of rapamycin was as effective as IP delivery at activating gene expression. Specifically, when mice were injected with rapamycin (1 mg/kg) either IP or IN the level of gene expression was the same (Figure 2) and the level of gene expression was similar to that conferred by the constitutive AAV9 vector (Figure 3). Interestingly we noted that luc expression in the nasal airway wanes with time (Figure 3) adding another important safety feature as it suggests that the positively transduced cells are being turned over with time.

Mice injected with vectors have been followed for more than 5 months and no adverse events have been noted.

### EXAMPLE 5 - Influenza Challenge Studies

Constructs based on specific human broadly neutralizing antibodies (Abs) have been cloned into highly efficient lung-directed AAV vectors and expressed under control of a chicken β-actin promoter. One of these constructs is based on CR6261, a broadly-neutralizing Ab isolated by Crucell (Holland), and another is based on FI6, a broadly-neutralizing antibody isolated by Humabs BioMed SA. Protein expression levels from both AAV -CB - antibody constructs have been confirmed *in vitro* by Western Blot and ELISA using a polyclonal anti-human IgG Ab.

For both the vector and the influenza challenge, BalbC mice weighing approximately 18-25 g were anesthetized with a mixture of ketamine/xylazine (70/7 mg/kg, injected intraperitoneally, IP). Using a clean mouse cage as a prop, a strip of surgical tape (1/4" width) is run across the mouth of the cage and secured. The mouse was then suspended by its dorsal incisors over the tape such that the nose tilted slightly upright. Its body weight was supported by placing gauze under the hind limbs. Using a sterile pipet tip (*e.g,*. P-20 Pipetman), a total volume of up to 30 µl of neuraminidase was instilled over 5 mins and 5-10 mins later 2x10¹¹ GC of either AAV2/9.CB.CR6261 or AAV2/9.CB.FI6 vector were administered slowly (over approximately 5 mins). Solution was instilled by placement of a small drop of vector solution just outside one of the nares allowing time for complete inhalation of the drop. Nares were alternated until all solution was applied. After solution administration was completed the mice were removed from the tape and allow to recover in a clean, dry cage.

The protective efficacy of AAV2/9-mediated anti-flu neutralizing antibody expression at the mucosal surface of the nose and lung of BalbC mice was demonstrated. Specifically, BalbC mice were given 50 µl of AAV2/9.CB.CR6261 or AAV2/9.CB.FI6 vector (2X10¹¹ genome copies, GC) intranasally (IN) 5-10 mins after mice were first given neuraminidase intranasally (IN). Fourteen days later mice were subjected to a lethal challenge IN with mouse-adapted PR8 influenza (H1N1) virus. All mice vaccinated with the AAV.CB.FI6 vector were fully protected and exhibited minimal weight loss, and 75% of mice vaccinated with the AAV2/9.CB.CR6261 vector were fully protected (Fig. 4).

### Example 6 - In Vivo Studies

For both the vector and the influenza challenge, female BalbC mice weighing approximately 18-25 g were anesthetized with a mixture of ketamine/xylazine (70/7 mg/kg, injected intraperitoneally). Using a clean mouse cage as a prop, a strip of surgical tape (1/4" width) was run across the mouth of the cage and secured. The mouse was then suspended by its dorsal incisors over the tape such that the nose tilted slightly upright. Its body weight was supported by placing gauze under the hind limbs. Using a sterile pipet tip (e.g. P-20 Pipetman), a total volume of up to 5µl of neuraminidase was instilled in each nare (when noted) and 5-10 mins later either AAV2/9.CB7.CR6261 or AAV2/9.CB7.FI6 vector in 50µl PBS were administered slowly (over approximately 5 mins). Solution was instilled by placement of a small drop of vector solution just outside one of the nares allowing time for complete inhalation of the drop. Nares were alternated until all solution was applied. After solution administration was completed the mice were removed from the tape and allow to recover in a clean, dry cage.

Similarly, for the influenza challenge 50µl of 10LD50 of PR8 or H3N2 was delivered as follows: using a clean mouse cage as a prop, a strip of surgical tape (1/4" width) was run across the mouth of the cage and secured. The mouse was then suspended by its dorsal incisors over the tape such that the nose tilted slightly upright. Its body weight was supported by placing gauze under the hind limbs. Using a sterile pipet tip (e.g. P-20 Pipetman), a total volume of up to 50µl PR8 or H3N2 in PBS was instilled in alternating nares. Solution was instilled by placement of a small drop of vector solution just outside one of the nares allowing time for complete inhalation of the drop. Nares were alternated until all solution was applied. After solution administration was completed the mice were removed from the tape and allow to recover in a clean, dry cage.

For targeted delivery to the nasopharynx the mouse was anaesthetized and using a_sterile pipet tip (*e.g.* P-20 Pipetman) 10µl of virus was delivered to each nostril while the mouse was horizontal and on its side (20µl total volume). Specifically, the mouse was placed horizontal on its right side and vector applied as a single bolus in its left nare. After 3 - 5 mins the mouse was placed horizontal on its left side and vector applied as a single bolus in its right nare. At the completion of solution administration the mouse was allowed to recover in a clean, dry cage.

### A. Evaluation of the minimal effective dose of AAV2/9 vector expressing FI6 antibody (Ab) following neuraminidase pretreatment.

BALB/c mice (n=5/group) were anaesthetized and dosed with increasing amounts of AAV2/9.CB7.FI6 (3x10⁹, 1x10¹⁰, 3x10¹⁰, 1x10¹¹ and 2x10¹¹ genome copies (GC)/mouse in 50µl PBS) to determine the minimal effective dose for the vectored FI6 Ab. In the same experimental setting, the protective efficacy of the FI6 Ab expressed in the lung following systemic administration of AAV2/9.CB7.FI6 was assessed. BALB/c mice (n=5/group) were dosed with decreasing amounts (1x10¹¹, 1x10¹⁰, 1x10⁹ and 1x10⁸ GC/mouse in 100µl PBS injected via the tail vein) of AAV2/9.CB7.FI6 was also assessed. Mice were challenged two weeks later with 10LD₅₀ of PR8 in 50µl PBS.

Intravenous delivery of AAV2/9.CB7.FI6 was not protective against influenza challenge at any of the doses studied. In stark contrast, AAV2/9.CB7.FI6 protected mice from the influenza challenge at doses of 1x10¹⁰, 3x10¹⁰, 1x10¹¹ and 2x10¹¹ GC/mouse (Figs 5A and 5B).

### B. Evaluation of the minimal effective dose of AAV2/9 vector expressing FI6 in the absence of neuraminidase pretreatment.

BALB/c mice (n=5/group) were dosed with decreasing amounts (3x10⁹, 1x10¹⁰, 1x10¹¹ GC/ mouse) of AAV2/9.CB7.FI6 to determine the minimal effective dose. Mice were challenged two weeks later with 10LD₅₀ of PR8. Mice given 3x10⁹ GC/mouse did not survive the challenge whereas mice given 1x10¹⁰ or 1x10¹¹GC/mouse in the absence of neuraminidase pretreatment survived.

### C. Evaluation of the protective efficacy of CR6261 or FI6 Abs expressed in the lung following systemic administration of AAV2/9.CB.CR6261 or AAV2/9.CB.FI6.

BALB/c mice (n=5/group) were dosed with 1x10¹¹ GC/mouse of AAV2/9.CB7.CR6261 or AAV2/9.CB7.FI6 vector given intranasally (IN) in 50 µl to target the lungs or in 20µl to target the nasopharynx. Mice were challenged two weeks later with 10LD₅₀ ofPR8. Mice given AAV2/9.CB7.FI6 that was targeted to lung or nose were fully protected upon challenge with influenza, whereas only the AAV2/9.CB7.CR6261 vector targeted to the lung was protective. Mice given AAV2/9.CB7.CR6261 targeted to nose, or irrelevant antibody AAV2/9.CB7.PG9 (control) targeted to the lung as well as naïve (no vector) mice did not survive the influenza challenge (Figs. 6A and 6B).

### D. Assessment of the protective efficacy of the AAV-Ab vector against a multi-influenza strain.

BALB/c mice (n=5/group) were injected IN with 1x10¹¹ GC of AAV2/9.CB7.CR6261 or AAV2/9.CB7.FI6 in 50 µl. Mice were challenged two weeks later with the A/Aichi/2/1968 x31 mouse adapted virus. As expected, only FI6 conferred universal protection against challenge with H3N2 (Fig. 7). Mice given AAV2/9.CB7.FI6 vector were fully protected whereas mice given AAV2/9.CB7.CR6261 vector and naïve mice lost significant weight and three out of five naïve mice had to be euthanized at day 8 due to a 28% weight loss. These studies are ongoing.

### E. In vivo assessment of the efficacy of the AAV-Ab vaccine.

The efficacy of the AAV-Ab constructs will be tested in BALB/c mice (n=10/group), using a dose of 2x10¹¹ genome copies (GC)/mouse shown to be effective in pilot studies. The vectors will be administered IN, in a total volume of 50µl, enabling targeting of the upper and lower respiratory tract. Neuraminidase (NA) will be administered concurrently to increase the availability of galactose, the receptor for AAV9, thereby improving the transduction of AAV9 as demonstrated in our lab by Bell and colleagues (J Clin Invest. 2011 Jun 1;121(6):2427-35). Control groups will include naïve mice given PBS instead of vector, with and without NA, as well as mice receiving an AAV2/9 vector expressing an unrelated antibody which should not impart protection against influenza. Two weeks after vector administration, all groups will be challenged with 100 LD₅₀ of three strains of H5N1 (HK/97, *A*/*Indonesia*/*5*/*2005*, Vietnam *2005) and H1N1* 1918 virus, inoculated IN in 50µl under animal biosafety level 4 (ABSL4) conditions. This dose of influenza causes a fatal decline in weight and mice will die. Symptoms and weights will be monitored daily, and any mice with ≥ 40% weight loss or exhibiting signs of distress will be euthanized. Organs, including lung, nasal turbinates, liver and spleen, will be collected to determine influenza viral load and to assess Ab expression by RT-qPCR. Serum, *bronchoalveolar* lavage fluid (BALF) and nasal lavage fluid (NLF) will also be collected at the time of necropsy. Any damage to the lung architecture, caused by influenza replication, will be evaluated by H&E staining of lung sections. Lung sections will also be immunostained for human IgG Ab expression. The experiment will be terminated 21 days post-challenge, after which any surviving mice will be killed to obtain tissue samples as described above.

### F. Evaluation of the kinetics of AAV-Ab vector-mediated protection against lethal challenge with influenza.

The kinetics of the AAV-Ab vaccine protection against influenza challenge will be assessed in BALB/c mice (n=10/group) by IN inoculating mice with AAV2/9 (2x10¹¹GC) expressing CR6261, FI6 or a control antibody. Groups of AAV vector-treated mice will be challenged with PR8 or H3N2 (X31 strain) at various time points post vector treatment, ranging from early (7 days) to late (90 days). Four different time points will be examined: days 7, 14, 28 and 3 months. The mice will be weighed daily for up to 21 days after the challenge and euthanized as required.

### G. Evaluation of the treatment efficacy of AAV-Ab given to mice following influenza exposure.

BALB/c mice (n=10/group) will be challenged IN with PR8 (or H3N2). At days 1, 2 or 4 post-influenza challenge vector-treated mice will be given 10¹¹ GC of AAV8-Ab intravenously. Mice will be monitored as specified in the Results section. Duration of the study is expected to be 4 weeks.

All publications, patents and patent applications, cited in this specification are incorporated herein by reference. While the invention has been described with reference to particularly preferred embodiments, it will be appreciated that modifications can be made without departing from the spirit of the invention.

The following are particular aspects of the invention:
1. A passive immunization regimen for an airborne pathogen, said regimen comprising specifically expressing anti-pathogen constructs in a subject's nasopharnyx cells by delivering to said cells a composition comprising an AAV viral vector comprising a nucleic acid sequence encoding an anti-pathogen construct operably linked to expression control sequences.
2. The regimen according to aspect 1, wherein said AAV vector comprises a regulatable promoter which directs expression of anti-pathogen construct following activation by a small molecule compound.
3. The regimen according to aspect 2, wherein the regulatable promoter is selected from the group consisting of a tet-on/off system, a tetR-KRAB system, a mifepristone (RU486) regulatable system, a tamoxifen-dependent regulatable system, a rapamycin - regulatable system, or an ecdysone-based regulatable system.
4. The regimen according to aspect 2, wherein said small molecule compound is rapamycin or a rapamycin analog.
5. The regimen according to aspect 2, wherein expression of the anti-pathogen construct is detectable in the nasopharynx of said subject within twenty-four hours following delivery of the small molecule compound.
6. The regimen according to aspect 2, wherein the small molecule compound is delivered intranasally.
7. The regimen according to aspect 6, wherein the small molecule compound is administered topically.
8. The regimen according to aspect 2, wherein the small molecule compound is delivered systemically.
9. The regimen according to any one of aspects 1 to 8, where said AAV viral vector transduces the subject's nasopharynx cells in the presence of high level serum-circulating AAV neutralizing antibodies.
10. The regimen according to any one of aspects 1 to 9, wherein the regimen comprises delivering a composition comprising an effective amount of the AAV viral vectors intranasally, such that a therapeutically effective amount is delivered to the nasopharynx in the absence of any therapeutically significant expression in the lung.
11. The regimen according to any one of aspects 1 to 10, wherein said airborne pathogen is a pathogenic virus and the anti-pathogen constructs are neutralizing antibody constructs directed against said virus.
12. The regimen according to aspect 11, wherein said neutralizing antibody construct neutralizes more than one subtype of said pathogenic virus.
13. The regimen according to aspect 11 or aspect 12, wherein said neutralizing antibody construct is selected from the group consisting of a full-length antibody, a single chain antibody, a Fab fragment, a univalent antibody, and an immunoadhesin.
14. The regimen according to aspect 13, wherein said neutralizing antibody construct is a monoclonal antibody.
15. The regimen according to aspect 12, wherein said pathogenic virus is selected from the group consisting of influenza, Ebola virus, and severe acute respiratory syndrome.
16. The regimen according to aspect 15, wherein said pathogenic virus is influenza A.
17. The regimen according to aspect 16, wherein said influenza A is selected from H1N1 and H3N2.
18. The regimen according to aspect 1, wherein said airborne pathogen is a bacterium and the anti-pathogen constructs are anti-microbial constructs directed against said bacteria or a pathogenic toxin thereof.
19. The regimen according to aspect 18, wherein said anti-pathogen construct is a neutralizing antibody construct against the protective antigen (PA) component of the toxin of *Bacillus anthracis.*
20. The regimen according to any one of aspects 1 to 19, wherein said regimen comprises delivering a combination of AAV vectors which comprise different anti-pathogen constructs.
21. The regimen according to any one of aspects 1 to 20, wherein said AAV vector comprises a AAV capsid selected from the group consisting of AAV1, AAV2, AAV3, AAV4, AAV5, AAV6, AAV6.2, AAV7, AAV8, AAV9, rh10, rh64R1, rh64R2 and rh8.

## Claims

1. A composition comprising a liquid carrier and an AAV viral vector comprising (i) an AAV capsid which targets nasal epithelial cells and (ii) a nucleic acid sequence encoding an anti-influenza neutralizing antibody construct operably linked to expression control sequences packaged in the AAV capsid, for use in passive immunization against influenza virus, wherein the composition is formulated for low volume intranasal administration of an effective amount of the AAV viral vector to the nasopharynx in the absence of any therapeutically significant expression in the lung, wherein the administration of the low volume restricts more than 95% of transduction of the AAV vector comprising the sequence encoding the neutralizing antibody construct to the nasal epithelium.

2. The composition according to claim 1, wherein said AAV vector comprises a regulatable promoter which directs expression of neutralizing antibody construct following activation by a small molecule compound, wherein the regulatable promoter is selected from a tet-on/off system, a tetR-KRAB system, a mifepristone (RU486) regulatable system, a tamoxifen-dependent regulatable system, a rapamycin-regulatable system, or an ecdysone-based regulatable system.

3. The composition according to claim 2, wherein said small molecule compound is rapamycin or a rapamycin analog.

4. The composition according to claim 3, wherein said neutralizing antibody construct neutralizes more than one subtype of influenza virus.

5. The composition according to claim 3 or claim 4, wherein said neutralizing antibody construct is selected from a full-length antibody, a single chain antibody, a Fab fragment, a univalent antibody, or an immunoadhesin.

6. The composition according to claim 5, wherein said neutralizing antibody construct is a monoclonal antibody.

7. The composition according to any one of claims 4 to 6, wherein said pathogenic virus is influenza A.

8. The composition according to claim 7, wherein said influenza A is selected from H1N1 or H3N2.

9. The composition according to any one of claims 1 to 8, wherein said AAV vector comprises an AAV capsid of AAV9.
